(19) Europäisches Patentamt / European Patent Office / Office européen des brevets

(11) **EP 1 739 891 B1**

(12) **EUROPEAN PATENT SPECIFICATION**

(45) Date of publication and mention
of the grant of the patent:
**12.11.2008 Bulletin 2008/46**

(51) Int Cl.:
*H04L 12/413* (2006.01)

(21) Application number: **05020929.5**

(22) Date of filing: **26.09.2005**

(54) **Transmission apparatus for reducing delay variance and related method**

Übertragungsvorrichtung zur Reduzierung der Varianz von Verzögerungen und zugehöriges Verfahren

Dispositif de transmission pour la réduction de variance de retard et procédé correspondant

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.06.2005 US 160522**

(43) Date of publication of application:
**03.01.2007 Bulletin 2007/01**

(73) Proprietor: **ZyXEL Communications Corporation Hsien**
**Hsin chu (TW)**

(72) Inventors:
• **Chen, Yih-Shen**
**c/o ZyXel Communications Corp.**
**Hsin-Chu Hsien (TW)**
• **Chang, Chung-Ju**
**c/o ZyXel Communications Corp.**
**Hsin-Chu Hsien (TW)**
• **Yen, Zhi-Ming**
**c/o Communications Corp.**
**Hsin-Chu Hsien (TW)**

(74) Representative: **Weber, Joachim**
**Hoefer & Partner**
**Patentanwälte**
**Pilgersheimer Strasse 20**
**81543 München (DE)**

(56) References cited:
**EP-A- 1 233 574          US-A1- 2003 161 340**

• YEH S-C ET AL: "Dynamic accommodation of the permission probability over the joint voice/data frame reservation multiple access" COMPUTER COMMUNICATIONS, ELSEVIER SCIENCE PUBLISHERS BV, AMSTERDAM, NL, vol. 22, no. 13, 25 August 1999 (1999-08-25), pages 1260-1265, XP004178586 ISSN: 0140-3664
• HYU-DAE KIM; WIE S; DONG-HO CHO: "A prioritized random access with discriminative power ramping step size" vol. 4, - 24 September 2000 (2000-09-24) pages 1751-1757, XP010524331 Vehicular Technology Conference, 2000. IEEE VTS Fall VTC 2000. 52nd Sept. 24-28, 2000, Piscataway, NJ, USA,IEEE ISBN: 0-7803-6507-0

**Description**

The invention relates to a transmission apparatus and related method for reducing delay variance according to the pre-characterizing clauses of claim 1 and claim 7.

Multiple access technology is popular is digital communication systems. For example, the Carrier Sense Multiple Access with Collision Avoidance (CSMA/CA) protocol is generally utilized to transmitting digital data in a communication channel, and provides a plurality of senders in the communication channel an equal opportunity to send data. To prevent the data sent by different senders from causing collisions, the CSMA/CA protocol adopts a Binary Exponential Backoff (BEB) algorithm. The key feature of the BEB algorithm is to delay the retransmission of a collided packet by a random time. As the number of collisions increase, the range of the random time increases. For example, after a sender S1 sends a packet P1 in the communication channel, the sender S1 listens to the communication channel to determine if the sent packet collided with a packet sent by another sender. If a collision is detected by the sender S1, the sender S1 will randomly selected a time from the set {0us, 51.2us} to retransmit the packet P1. If 0us is selected, the sender S1 retransmits the packet P1 immediately. If the 51.2us is selected, the sender S1 will retransmit the packet P1 51.2us later. If the packet P1 collided a second time with a packet sent by another sender then the sender S1 will randomly select a time from the set {0us, 51.2us, 102.4us, and 153.6us} to retransmit the packet P1. The number of the elements of the set increases exponentially. In the same manner, if the packet P1 is collided for three times, the sender S1 randomly selects a time from the set {0us, 51.2us, 102.4us, 153.6us, 204.8us, 256us, 307.2us, and 358.4us} to retransmit the packet P1. After the packet P1 is retransmitted more than ten times, the range of randomly selected time values (i.e., the set of time) remains. The number of collisions will increase as more senders utilize the communication channel to transmit data. Because of the collision, some data are delay. However, some other data are quickly received by the receiver if no collision occurs. As a result, the delay variance of those data increases.

Yeh, S.-C. et al., "Dynamic accommodation of the permission probability over the joint voice/data frame reservation multiple access" discloses some schemes for dynamic accommodation of the permission probability based on the frame reservation multiple access protocol, a variant of PRMA, where the base station broadcasts the acknowledgments for all the slots in a frame which occurs at the end of the frame. The focus of the disclosed schemes is based on a protocol called adaptive permission reservation (APR), which implements an algorithm to estimate and adjust the sending permission according to the number of contending terminals for multiplexing voice traffic in wireless communications. Yeh et al. suggest to vary the permission probability according to the available slots between one frame and the next and/or to assign the permission probability individually by the contending number of retrials for each active terminal. In particular, in the first proposed scheme, the value of the permission probability stays constant throughout a frame, but can be accommodated from one frame to the next according to a particular formula. The second scheme presents a higher permission probability to a user who has a large contending number of retrials, i.e. the user has failed many times due to collision, and a lower permission probability to a user with a smaller contending number of retrials.

Further, EP 1 233 574 A2 discloses a unified channel access for supporting the quality of service in a local area network. In particular, the shared communication is captured, a recipient is permitted to transmit and the shared communications medium is recaptured after the recipient transmitted. The method also includes to wait for the shared communications medium to become idle prior to capturing the shared communications medium. After a frame is transmitted, the contending station waits for an acknowledgment and if the contending station does not receive an acknowledgment, then the transmission was a failure and the contending station may attempt to re-transmit the frame. The re-transmission of the failed frame is treated the same way as if the re-transmission was an initial attempt at transmitting a frame.

Still further, US 2003/0161340 A1 discloses a method and system for optimally serving stations on wireless LANs using a controlled contention/resource reservation protocol of the IEEE 802.11E standard. It is described that permission probability would ideally be used to limit the number of contenders so as to guarantee a reasonable probability of success within one controlled contention interval. However, on the other hand it is explicitly stated that an operation without permission probability is possible and permission probability is not required.

Moreover, the document HYU-DAE KIM; WIE S; DONG-HO CHO: "A prioritized random access with discriminative power ramping step size" vol. 4, - 24 September 2000 (2000-09-24) pages 1751-1757, XP010524331 Vehicular Technology Conference, 2000. IEEE VTS Fall VTC 2000. 52$^{nd}$ Sept. 24-28, 2000, Piscataway, NJ, USA, IEEE ISBN: 0-7803-6507-0, describes that in mobile-communication-systems a power ramping is performed which is the increase of the power level of a burst for the purpose of obtaining the higher access probability in random access. According to this scheme, if an access attempt fails, the transmission of the burst is retried with a higher power level and such a power ramping is continued until it is successful. Therefore, the increase of the access probability after an access failure is described.

Since the digital communication systems for executing real time applications have gained more popularity in past recent years. People can download static information from the Ethernet and enjoy movies or songs on-line. For example, when an end user utilizes a terminal equipment, such as a personal computer, to receive and then play in real time a movie, the terminal equipment must establish a connection with a server providing the real time service. Next, the server will transmits a series of data D1, D2...Dn to the personal computer. The personal computer reconstructs the received data D1, D2...Dn to form a frame F1, and then displays the frame F1 on the monitor of the personal computer. In the same manner, before the monitor shows the next frame F2, the personal computer must successfully receive the data Dn+1, Dn+2, ...D2n from the server, and reconstruct the data Dn+1, Dn+2, ...D2n to form the frame F2. In an ideal situation, the sequentially transmitted data from the server are received by the personal computer one by one. In other words, the delay times of every data from the server to the personal computer are identically equal. However, since the condition of the communication channel is very dynamic and changes with time, the delay times of every data are different. For example, when transmitting the data D1 and D2, if the communication channel is clear, the data D1, D2 is received by the personal computer without retransmitting. When transmitting the data D3, D4, if the communication channel is noisy (i.e., too many senders in the communication channel), the data D3, D4 may be retransmitted several times because of collisions. As a result, the delay times of the data D3, D4 is longer than the delay times of the data D1, D2. Please note, as the delay variance of data increases the user will feel more uncomfortable as the frames will be displayed with unequal speed. To help alleviate this problem, the personal computer requires a larger buffer to store more data. The larger buffer is necessary because the digital communication system adopting the CSMA/CA protocol and BEB algorithm has no mechanism and does not attempt to solve the problem mentioned above. As a result, the amount of computation, the required system resources, and the power consumed by the terminal equipment all increase accordingly.

Besides, the traditional BEB algorithm is unfair. For example, the BEB algorithm may be favorable to the station just join the LAN. Consequently, many methods, such as the decentralized delay fluctuation control (DDFC) mechanism, are disclosed to solve this problem. However, these methods also have some drawbacks. Take the DDFC mechanism as an example, the DDFC mechanism may increase the collision probability and reduces the effective throughput consequently. As a result, a fair method capable of reducing the delay variance is necessary.

This in mind, the present invention aims at providing a further transmission apparatus applied in a digital communication system and related method to reduce the delay variance.

This is achieved by a method according to claim 1 and a transmission apparatus according to claim 6. The dependent claims pertain to corresponding further developments and improvements.

As will be seen more clearly from the detailed description following below, the claimed method applied in a communication system for reducing the delay variance of a plurality of data is disclosed. The plurality of data is transmitted from a sender to a receiver in the communication system. The method includes: assigning a permission probability to the sender; and before a data is to be sent to the receiver from the sender, determining if the sender is allowed to transmit the data according to the permission probability, and if the sender is not allowed to transmit the data according to the permission probability, increasing the permission probability assigned to the sender and then driving the sender to re-transmit the data by going back to the previous determining step.

As will be seen more clearly from the detailed description following below, the claimed transmission apparatus applied in a communication system for reducing the delay variance of a plurality of data is disclosed. The plurality of data is transmitted in the communication system. The transmission apparatus includes: a permission probability generating module adapted to generate a permission probability; and a transmission module, electrically connected to the permission probability generating module, adapted to transmit a data according to the permission probability; wherein the transmission module is adapted to determine if it is allowed to transmit the data according to the permission probability before a data is to be sent by the transmission module, wherein the permission probability generating module is adapted to increase the permission probability and then drive the transmission module to re-transmit the data by going back to the previous determining step if the transmission module is not allowed to transmit the data according to the permission probability.

In the following, the invention is further illustrated by way of example, taking reference to the accompanying drawings. Thereof

Fig. 1 is a flow chart of the method for reducing the delay variance according to a preferred embodiment of the present invention, and

Fig. 2 is a schematic diagram of the transmission apparatus according to a preferred embodiment of the present invention.

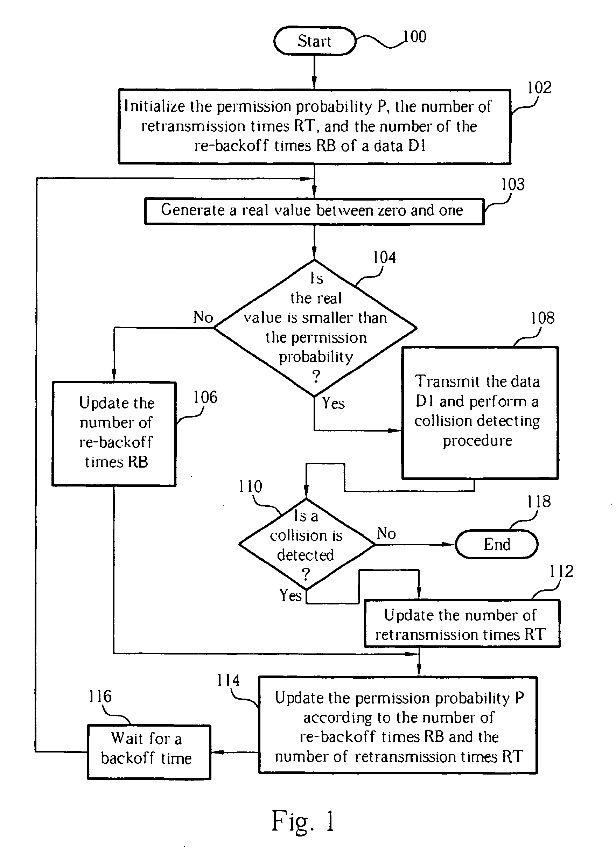

Please refer to Fig. 1. Fig. 1 is a flow chart of the method for reducing the delay variance according to a preferred embodiment of the present invention. In the preferred embodiment, the sender utilizing the method transmits data to a receiver according to the Carrier Sense Multiple Access with Collision Avoidance (CSMA/CA) protocol. The sender and the receiver are in a digital communication systems, such as a WLAN system or LAN system. The method comprises the following steps:

Step 100:    Start.

Step 102:    Assign the permission probability P of a data D1 to an initial value P0, and assign the number of retransmission times RT of the data D1, the number of the re-backoff times RB of the data D1, and a backoff time of the data D1 to zero.

Step 103:    Generate a real value between zero and one.

Step 104:    If the real value is smaller than the permission probability then proceed to step 108; otherwise, proceed to step 106.

Step 106:    Update the number of re-backoff times RB by adding the number of re-backoff times RB to one, and proceed to step 114.

Step 108:    Transmit the data D1 and then perform a collision detecting procedure to detect if a collision happens to the data D1.

Step 110:    If the collision is detected, proceed to step 112; otherwise, proceed to step 118.

Step 112:    Update the number of retransmission times RT by adding the number of retransmission times RT to one, and update the backoff time according to the BEB algorithm.

Step 114:    Update the permission probability P according to the number of re-backoff times RB and the number of retransmission times RT.

Step 116:    Wait for the backoff time, and proceed to step 103.

Step 118:    End.

Firstly, as the sender prepares to transmit a data D1, the sender initializes the permission probability P, the number of retransmission times RT, and the number of the re-backoff time RB of the data D1 (Step 102). According to the present embodiment, the initial value P0 of the permission probability P is 0.5, and the initial values of the number of retransmission times RT and the number of the re-backoff time RB are both zero. Please note that the initial values mentioned above are not limited by the present embodiment. Next, the sender determines if it is allowed to transmit the data D1 according to the permission probability P (Steps 103, 104) . In the preferred embodiment, the sender generates a real value between zero and one. If the real value is smaller than the permission probability, the sender is allowed to transmit the data D1; otherwise, the sender is not allowed to transmit the data D1.
If the sender is not allowed to transmit the data D1, the sender will update the number of re-backoff times RB by adding the number of re-backoff times RB to one (step 106) according to the present embodiment, and the backoff time of the data D1 remains. If the sender is allowed to transmit the data D1, the sender will send the data D1 and perform a collision detecting procedure (108). If no collision is detected, the data D1 is transmitted to the receiver successfully. That is the sender will prepare to send the next data D2, and performs the method again. If a collision is detected, the sender will update the number of retransmission times RT by adding the number of retransmission times RT to one, and update the backoff time according to the BEB algorism (step 112). Next, the sender calculates a new permission probability P according to the number of re-backoff times RB and the number of retransmission times RT. According to the preferred embodiment, the operation of calculating the permission probability P is represented by the following equation:

$$P = P0 + \frac{1 - P0}{BS_{MAX}} \cdot (RT + \frac{RB}{RB_{MAX} + 1}) \qquad\qquad \text{Equation (1)}$$

$BS_{MAX}$ denotes the maximum backoff stage of the BEB algorithm. In the CSMA/CA protocol, the maximum backoff stage is ten. $RB_{MAX}$ denotes the maximum of the number of the re-backoff times. According to the Equation (1), the permission probability P increases as the number of re-backoff times RB or the number of retransmission times RT increase. As the calculated permission probability P is greater than one, the permission probability P is determined to be one. In Equation (1), as the number of the retransmission of a specific data increases, the permission probability P of the specific data is higher. In other words, when the specific data is transmitted at a first time, the permission probability P of a specific data is the lowest. As a result, the delay times of a plurality of data are balanced through utilizing the permission probability and the delay variance is reduced at the same time. After the permission probability is calculated, the sender waits for the backoff time then determines if it is allowed to transmit the data D1 according to the updated permission probability P. Please note that the operation of determining the backoff time is well known by those skilled in the art therefore the related description is omitted for the sake of brevity. It should be noted that the method of generating the permission probability P is not limited to the Equation (1). Other methods for adjusting the permission probability P according to the number of re-backoff times RB or the number of retransmission times RT are covered by the claimed invention.

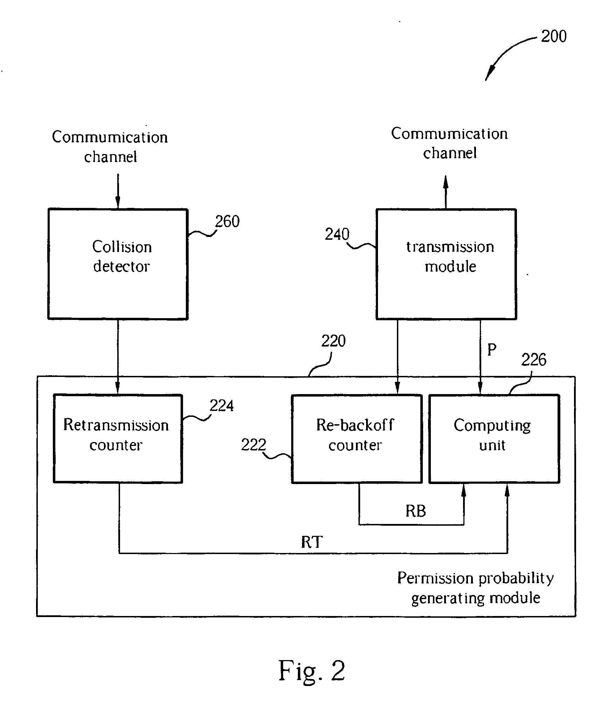

Please refer to Fig. 2. Fig. 2 is a schematic diagram of the transmission apparatus 200 capable of reducing the delay variance according to a preferred embodiment of the present invention. According to the preferred embodiment, the transmission apparatus 200,transmits data according to the Carrier Sense Multiple Access with Collision Avoidance (CSMA/CA) protocol, and is applied in a digital communication system, such as WLAN or LAN system. The transmission apparatus 200 comprises a permission probability generating module 220, a transmission module 240, and a collision detector 260. In the beginning, the permission probability generating module 220 generates a permission probability P to the transmission module 240. Next, the transmission module 240 determines that if it is allowed to transmit a data D1 according to the permission probability P. If it is not allowed to transmit the data D1, the permission probability generating module 220 will adjust the permission probability P higher. If the data D1 is transmitted by the transmission module 240, the collision detector 260 will perform a collision detecting procedure to detect if a collision happens to the data D1. Next, if a collision is detected, the permission probability generating module 220 will adjust the permission probability P higher. If no collision is detected (i.e., the data D1 is transmitted successfully), the permission probability generating module 220 will reset the permission probability P to an initial value P0 applied to transmission of a next data D2.

According to the preferred embodiment, the permission probability generating module 220 further comprises a re-backoff counter 222, a retransmission counter 224, and a computing unit 226. The re-backoff 222 is utilized to count the number of the re-backoff times RB of a specific data when the specific data is not allowed to be transmitted according to the permission probability. The retransmission counter 224 is utilized to count the number of the retransmission times RT of the specific data when a collision is detected. The computing unit 226 calculates the permission probability P of the specific data according to the number of the retransmission times RT and the number of the re-backoff times RB. Please note that operation of the computing unit 226 are detailed in the Equation (1) according to the preferred embodiment. So the detailed description of the operation of the computing unit 226 is omitted.

It should be noted that the method and apparatus capable of reducing the delay variance are not limited to combining with the CSMA/CA protocol. According to the claimed invention, the method and apparatus capable of reducing the delay variance may be combined with another kinds of multiple access protocols.

Compared with the prior art, the permission probability generating module generates a permission probability to determine if a data is allowed to be transmitted according to the present invention. Since the data, which is retransmitted more times or suffered collision more times, has higher permission probability, the probability of the data's successful transmission increases. Hence, the delay times of a plurality of data are balanced resulting in the delay variance being reduced accordingly.

## Claims

1. A method for reducing the delay variance of a plurality of data transmitted from a sender to a receiver in a digital communication system, the method comprising the steps:

    assigning a permission probability to the sender (102); and
    before a data is to be sent to the receiver from the sender, determining if the sender is allowed to transmit the data according to the permission probability (104);

    **characterized by**:

    if the sender is not allowed to transmit the data according to the permission probability, increasing the permission probability assigned to the sender (106, 114) and then driving the sender to re-transmit the data by going back to the previous determining step.

2. The method of claim 1, further **characterized by**:

if the data is transmitted to the receiver by the sender according to the permission probability (108), performing a collision detecting procedure to detect if a collision happens to the data (110); and

if the collision is detected by the collision detecting procedure, increasing the permission probability assigned to the sender (112, 114) and then driving the sender to re-transmit the data.

3. The method of claim 2, further **characterized by**:

if the collision is not detected by the collision detecting procedure, resetting the permission probability to an initial value applied to transmission of a next data.

4. The method of claim 1, **characterized in that** the data is transmitted according to a Carrier Sense Multiple Access with Collision Avoidance (CSMA/CA) protocol.

5. The method of claim 1, **characterized in that** the digital communication system is a WLAN.

6. A transmission apparatus (200) for reducing the delay variance of a plurality of data transmitted to a receiver in a digital communication system, the transmission apparatus (200) comprising:

a permission probability generating module (220) adapted to generate a permission probability; and

a transmission module (240), electrically connected to the permission probability generating module (220), adapted to transmit a data according to the permission probability;

wherein the transmission module (240) is adapted to determine if it is allowed to transmit the data according to the permission probability before a data is sent by the transmission module (240);

**characterized in that** the permission probability generating module (220) is adapted to increase the permission probability and then drive the transmission module (220) to re-transmit the data by going back to the previous determining step if the transmission module (240) is not allowed to transmit the data according to the permission probability.

7. The transmission apparatus (200) of claim 6, further **characterized by**:

a collision detector (260), adapted to perform a collision detecting procedure to detect if a collision happens to the data when the data is transmitted according to the permission probability;

wherein the permission probability generating module (220) is adapted to increase the permission probability and then drive the sender to re-transmit the data if the collision is detected by the collision detector (260).

8. The transmission apparatus (200) of claim 7, **characterized in that** the permission probability generating module (220) is adapted to reset the permission probability to an initial value applied to transmission of a next data if no collision is detected by the collision detector (260).

9. The transmission apparatus (200) of claim 6, **characterized in that** the data is transmitted according to a carrier sense multiple access with collision avoidance (CSMA/CA) protocol.

10. The transmission apparatus (200) of claim 6, **characterized in that** the digital communication system is a WLAN.

**Patentansprüche**

1. Verfahren zur Verringerung der Verzögerungsvarianz einer Mehrzahl von Daten, die in einem digitalen Kommunikationssystem von einem Sender an einen Empfänger übertragen werden, wobei das Verfahren die Schritte umfasst:

Zuordnen einer Erlaubniswahrscheinlichkeit für den Sender (102); und

Ermitteln, ob der Sender die Daten gemäß der Erlaubniswahrscheinlichkeit (104) übertragen darf, bevor Daten von dem Sender an den Empfänger geschickt werden sollen; **dadurch gekennzeichnet, dass**

wenn der Sender die Daten gemäß der Erlaubniswahrscheinlichkeit nicht übertragen darf, die dem Sender (106, 114) zugeordnete Erlaubniswahrscheinlichkeit erhöht wird, und der Sender dann veranlasst wird, die Daten nochmals zu übertragen, indem zu dem vorherigen Ermittlungsschritt zurückgegangen wird.

**2.** Verfahren gemäß Anspruch 1, ferner **dadurch gekennzeichnet, dass,**
wenn die Daten durch den Sender gemäß der Erlaubniswahrscheinlichkeit (108) an den Empfänger übertragen werden, eine Kollisionserfassungsprozedur durchgeführt wird, um zu erfassen, ob die Daten (110) von einer Kollision betroffen sind; und

wenn die Kollision durch die Kollisionserfassungsprozedur erfasst wird, die dem Sender (112, 114) zugeordnete Erlaubniswahrscheinlichkeit erhöht wird, und dann der Sender veranlasst wird, die Daten nochmals zu übermitteln.

**3.** Verfahren gemäß Anspruch 2, **dadurch gekennzeichnet, dass,**
wenn die Kollision durch die Kollisionserfassungsprozedur nicht erfasst wird, die Erlaubniswahrscheinlichkeit auf einen anfänglichen Wert zurückgesetzt wird, der auf die Übertragung von nachfolgenden Daten angewandt wird.

**4.** Verfahren gemäß Anspruch 1, **dadurch gekennzeichnet, dass** die Daten gemäß einem "Mehrfachzugriff mit Trägerprüfung und Kollisionserkennung (CSMA/CA)"-Protokoll übertragen werden.

**5.** Verfahren gemäß Anspruch 1, **dadurch gekennzeichnet, dass** das digitale Kommunikationssystem ein WLAN ist.

**6.** Übertragungsvorrichtung (200) zur Verringerung der Verzögerungsvarianz einer Mehrzahl von Daten, die in einem digitalen Kommunikationssystem an einen Empfänger übertragen werden, wobei die Übertragungsvorrichtung (200) umfasst:

ein Erlaubniswahrscheinlichkeit-Erzeugungsmodul (220), das dafür ausgelegt ist, eine Erlaubniswahrscheinlichkeit zu erzeugen; und
ein mit dem Erlaubniswahrscheinlichkeit-Erzeugungsmodul (220) elektrisch verbundenes Übertragungsmodul (240), das dafür ausgelegt ist, Daten gemäß der Erlaubniswahrscheinlichkeit zu übertragen;

wobei das Übertragungsmodul (240) ausgelegt ist, zu ermitteln, ob es erlaubt ist, die Daten gemäß der Erlaubniswahrscheinlichkeit zu übertragen, bevor Daten durch das Übertragungsmodul (240) gesendet werden;
**dadurch gekennzeichnet, dass** das
Erlaubniswahrscheinlichkeit-Erzeugungsmodul (220) dafür ausgelegt ist, die Erlaubniswahrscheinlichkeit zu erhöhen, und dann das Übertragungsmodul (220) zu veranlassen, die Daten nochmals zu übertragen, indem zu dem vorherigen Ermittlungsschritt zurückgegangen wird, wenn das Übertragungsmodul (240) die Daten gemäß der Erlaubniswahrscheinlichkeit nicht übertragen darf.

**7.** Übertragungsvorrichtung (200) gemäß Anspruch 6, ferner **gekennzeichnet durch**:

einen Kollisionsdetektor (260), der ausgelegt ist, eine Kollisionserfassungsprozedur durchzuführen, um zu erfassen, ob die Daten von einer Kollision betroffen sind, wenn die Daten gemäß der Erlaubniswahrscheinlichkeit übertragen werden;
wobei das Erlaubniswahrscheinlichkeit-Erzeugungsmodul (220) dafür ausgelegt ist, die Erlaubniswahrscheinlichkeit zu erhöhen und dann den Sender zu veranlassen, die Daten nochmals zu übertragen, wenn die Kollision **durch** den Kollisionsdetektor (260) erfasst wird.

**8.** Übertragungsvorrichtung (200) gemäß Anspruch 7, **dadurch gekennzeichnet, dass** das Erlaubniswahrscheinlichkeit-Erzeugungsmodul (220) dafür ausgelegt ist, die Erlaubniswahrscheinlichkeit auf einen anfänglichen Wert zurückzusetzen, der auf die Übertragung von nachfolgenden Daten angewandt wird, wenn durch den Kollisionsdetektor (260) keine Kollision erfasst wird.

**9.** Übertragungsvorrichtung (200) gemäß Anspruch 6, **dadurch gekennzeichnet, dass** die Daten gemäß einem "Mehrfachzugriff mit Trägerprüfung und Kollisionserkennung (CSMA/CA)"-Protokoll übertragen werden.

**10.** Übertragungsvorrichtung (200) gemäß Anspruch 6, **dadurch gekennzeichnet, dass** das digitale Kommunikationssystem ein WLAN ist.

**Revendications**

**1.** Procédé pour réduire la variation de retard d'une pluralité de données transmises d'un émetteur à un récepteur dans un système de communication numérique, le procédé comprenant les étapes suivantes :

attribuer une probabilité de permission à l'émetteur (102) ; et

avant que des données soient envoyées au récepteur en provenance de l'émetteur, déterminer si l'émetteur est autorisé à transmettre les données selon la probabilité de permission (104) ; **caractérisé par** :

si l'émetteur n'est pas autorisé à transmettre les données selon la probabilité de permission, augmenter la probabilité de permission attribuée à l'émetteur (106, 114) et amener ensuite l'émetteur à retransmettre les données en retournant à l'étape de détermination précédente.

2. Procédé selon la revendication 1, **caractérisé en outre par** :

si les données sont transmises au récepteur par l'émetteur selon la probabilité de permission (108), exécuter une procédure de détection de collision afin de détecter si une collision se produit sur les données (110) ; et si la collision est détectée par la procédure de détection de collision, augmenter la probabilité de permission attribuée à l'émetteur (112, 114) et amener ensuite l'émetteur à retransmettre les données.

3. Procédé selon la revendication 2, **caractérisé en outre par** :

si la collision n'est pas détectée par la procédure de détection de collision, remettre la probabilité de permission à une valeur initiale appliquée à la transmission de données suivantes.

4. Procédé selon la revendication 1, **caractérisé en ce que** les données sont transmises selon un protocole d'accès multiple avec écoute de porteuse et évitement de collision (CSMA/CA).

5. Procédé selon la revendication 1, **caractérisé en ce que** le système de communication numérique est un WLAN.

6. Appareil de transmission (200) pour réduire la variation de retard d'une pluralité de données transmises à un récepteur dans un système de communication numérique, l'appareil de transmission (200) comprenant :

un module de génération de probabilité de permission (220) adapté pour générer une probabilité de permission ; et

un module de transmission (240), connecté électriquement au module de génération de probabilité de permission (220), adapté pour transmettre des données selon la probabilité de permission ;

dans lequel le module de transmission (240) est adapté pour déterminer s'il est autorisé à transmettre les données selon la probabilité de permission avant que des données soient envoyées par le module de transmission (240) ;

**caractérisé en ce que** le module de génération de probabilité de permission (220) est adapté pour augmenter la probabilité de permission et amener ensuite le module de transmission (240) à retransmettre les données en retournant à l'étape de détermination précédente si le module de transmission (240) n'est pas autorisé à transmettre les données selon la probabilité de permission.

7. Appareil de transmission (200) selon la revendication 6, **caractérisé en outre par** :

un détecteur de collision (260), adapté pour exécuter une procédure de détection de collision afin de détecter si une collision se produit sur les données lorsque les données sont transmises selon la probabilité de permission ;

dans lequel le module de génération de probabilité de permission (220) est adapté pour augmenter la probabilité de permission et amener ensuite l'émetteur à retransmettre les données si la collision est détectée par le détecteur de collision (260).

8. Appareil de transmission (200) selon la revendication 7, **caractérisé en ce que** le module de génération de probabilité de permission (220) est adapté pour remettre la probabilité de permission à une valeur initiale appliquée à la transmission de données suivantes si aucune collision n'est détectée par le détecteur de collision (260).

9. Appareil de transmission (200) selon la revendication 6, **caractérisé en ce que** les données sont transmises selon un protocole d'accès multiple avec écoute de porteuse et évitement de collision (CSMA/CA).

10. Appareil de transmission (200) selon la revendication 6, **caractérisé en ce que** le système de communication numérique est un WLAN.

Start ~100

Initialize the permission probability P, the number of retransmission times RT, and the number of the re-backoff times RB of a data D1 — 102

Generate a real value between zero and one — 103

Is the real value is smaller than the permission probability ? — 104

No

Update the number of re-backoff times RB — 106

Yes

Transmit the data D1 and perform a collision detecting procedure — 108

Is a collision is detected ? — 110

No

End — 118

Yes

Update the number of retransmission times RT — 112

Update the permission probability P according to the number of re-backoff times RB and the number of retransmission times RT — 114

Wait for a backoff time — 116

Fig. 1

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1233574 A2

- US 20030161340 A1

**Non-patent literature cited in the description**

- **HYU-DAE KIM ; WIE S ; DONG-HO CHO.** *A prioritized random access with discriminative power ramping step size,* 24 September 2000, vol. 4, 1751-1757